# EUROPEAN PATENT SPECIFICATION

(11) **EP 0 952 144 B1**
(45) Date of publication and mention of the grant of the patent: **18.06.2003**
(21) Application number: 98420069.1
(22) Date of filing: 20.04.1998
(51) Int. Cl.: C07C 255/66, C07C 253/30, C07D 213/77

(54) **Processes for preparing pesticidal intermediates**
Verfahren zur Herstellung von pestiziden Zwischenprodukten
Procédés pour la préparation d'intermédiaires pesticides

(43) Date of publication of application: 27.10.1999
(73) Proprietor: Bayer Agriculture Limited, Ongar, Essex CM5 0HW (GB)
(72) Inventor: Ancel, Jean Erick, 69230, Saint-Genis-Laval (FR)
(74) Representative: Mérigeault, Shona

(56) References cited:
- WO-A-97/32843
- DE-A- 3 612 940
- CHEMICAL ABSTRACTS, vol. 53, no. 5, 1959 Columbus, Ohio, US; abstract no. 5163h, MURAHASHI: "The synthesis and some chemical properties" page 5163; XP002079282 & NIPPON KAGAKU ZASSHI, vol. 77, 1956, pages 1689-1692,
- JACHAK ET AL.: "Synthesis with nitriles. XCI. Cyanoacetaldehyde - new synthetic applications of an old compound" MONATSHEFTE FÜR CHEMIE, vol. 124, no. 2, 1993, pages 199-207, XP002079281

## Description

This invention relates to novel processes for preparing intermediates (particularly 2-(arylhydrazino)succinonitrile compounds and 3-(arylhydrazono)propionitrile derivatives) useful in the preparation of pesticides.

European Patent Publication Nos. 0295117 and 0234119 describe the preparation of pesticidally active phenylpyrazole compounds and of 5-amino-1-aryl-3-cyanopyrazole intermediate compounds used in their synthesis.

Various methods for preparing these compounds are known. The present invention seeks to provide improved or more economical methods for the preparation of pesticides and the intermediate compounds useful in preparing them.

German Patent Publication No.3612940 discloses the preparation of 5-amino-1-arylpyrazole derivatives of general formula: 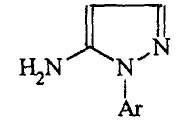 wherein Ar represents substituted phenyl or pyridyl, which can be used as intermediates in the preparation of compounds possessing herbicidal or pesticidal properties, by the reaction of arylhydrazine hydrochloride salts with formylacetonitrile sodium salt of formula:

NaOCH=CH-CN

to give hydrazone compounds of general formula:

Ar-NH-N=CH-CH₂-CN

wherein Ar is as hereinbefore defined; which are then cyclised in the presence of a base.

Chem. Abstr, vol 53, No. 5, 1959, 5163h describes the preparation of cyanoacetylene which, on reaction with methanol (MeOH) or ethanol (EtOH), give (MeO)₂ CHCH₂CN or (EtO)₂ CHCH₂CN, each of which gave the same phenylhydrazone on reaction with 2,4-dinitrophenylhydrazine sulphate. The phenylhydrazone reaction described is a routine characterisation test for the presense of an aldehyde or ketone group. The 2,4-dinitrophenyl derivative is always used for characterisation purposes: the two nitro groups exert a marked effect on the substituent attached at the 1-position. This renders uncertain any prediction of the reaction in the absence of nitro: nitro groups are not present in the compounds prepared by the process of the present application.

However it may be desirable to obtain the hydrazone compounds in a pure form useful for their further conversion into pesticides. Known procedures may result in the formation of hydrazones which are contaminated with the cyclised 5-amino-1-arylpyrazole product.

The present applicants have surprisingly discovered a novel process for the preparation of the hydrazone compounds without cyclisation occurring. The hydrazone compounds may then be used either to provide a new method to prepare the 5-amino-1-arylpyrazole compounds, or in a novel process which involves addition of a cyanide to provide 2-(arylhydrazino)succinonitrile derivatives which may be further processed to provide important 5-amino-1-aryl-3-cyanopyrazole compounds which are valuable intermediates for the preparation of pesticides.

The present invention accordingly provides a process (A) for the preparation of a compound of formula (I): 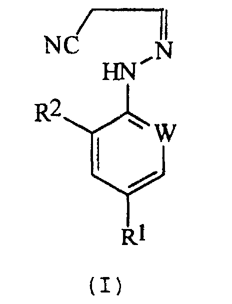 wherein
W represents nitrogen or -CR³;
R¹ represents halogen, haloalkyl (preferably trifluoromethyl), haloalkoxy (preferably trifluoromethoxy), R⁴S(O)ₙ-, or -SF₅;
R² represents hydrogen or halogen (for example chlorine or bromine);
R³ represents halogen (for example chlorine or bromine);
R⁴ represents alkyl or haloalkyl; and
n represents 0,1 or 2; which process comprises the reaction of a compound of formula (II) : 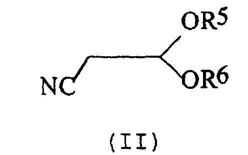
wherein R⁵ and R⁶ independently represent alkyl or together represent an alkylene chain containing two or three carbon atoms, with an acid addition salt of an arylhydrazine compound of formula (III): 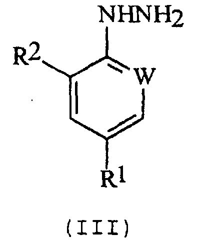 wherein R¹, R² and W are as hereinbefore defined. Compounds of formula (I) may exist as a mixture of syn and anti isomers.

Unless otherwise specified in the present specification 'alkyl' means straight- or branched- chain alkyl having from one to six carbon atoms (preferably one to three). Unless otherwise specified 'haloalkyl' and 'haloalkoxy' are straight- or branched- chain alkyl or alkoxy respectively having from one to six carbon atoms (preferably one to three) substituted by one or more halogen atoms selected from fluorine, chlorine or bromine.

Generally R⁵ and R⁶ in formula (III) represent the same alkyl group, preferably methyl or ethyl.

The acid addition salts of the compounds of formula (III) are preferably the salts formed from strong acids such as mineral acids, for example sulphuric acid, or preferably hydrochloric acid. Generally the salts are preformed but may optionally be generated in situ. The reaction may be conducted in a polar or a non-polar solvent in the presence of water. Examples of polar solvents include water; alcohols such as methanol or ethanol; nitriles such as acetonitrile; N-methylpyrrolidone or sulphoxides such as dimethyl sulphoxide. Examples of non-polar solvents include chlorinated hydrocarbons, preferably carbon tetrachloride; and hydrocarbons such as cyclohexane. The reaction temperature is generally from 20°C to 100°C, preferably from 50°C to 90°C. Equimolar amounts of the compounds of formula (II) and (III) are generally employed. The amount of water which may be present is from a catalytic amount to a large excess.

In formulae (I) and (III) and in the formulae depicted hereinafter, preferred values of the symbols are as follows:-
R¹ represents haloalkyl (preferably trifluoromethyl), haloalkoxy (preferably trifluoromethoxy) or -SF₅;
W represents -CR³; and R³ represents halogen;

A most preferred compound of formula (I) is 3-(2,6-dichloro-4-trifluoromethylphenylhydrazono)propionitrile.

Compounds of formula (II) and (III) are generally known in the literature.

The process of the invention is characterised by a number of advantages. Thus, it seeks to enable 3-arylhydrazono-propionitrile compounds of formula (I) to be obtained in high yield from readily available starting materials. Furthermore the reaction can be very simple and economical to perform, and product isolation is very straightforward. Furthermore the compounds of formula (I) can be obtained without cyclisation occurring.

According to a further feature of the present invention there is provided a process (B) for the preparation of a compound of formula (I), which comprises the reaction of a compound of formula (IV) : 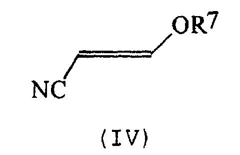 wherein R⁷ represents alkyl (preferably methyl or ethyl), with a compound of formula (III), wherein R¹, R² and W are as hereinbefore defined. The reaction conditions which are generally employed are the same as those used for the above preparation of a compound of formula (I) from the reaction of a compound of formula (II) with a compound of formula (III).

Compounds of formula (IV) are generally known in the literature.

According to a further feature of the present invention there is provided a process (C) for the preparation of a compound of formula (V) : 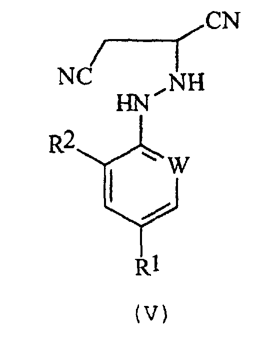 wherein R¹, R² and W are as hereinbefore defined; which process comprises the reaction of a compound of formula (I) wherein R¹, R² and W are as hereinbefore defined, with a source of hydrogen cyanide.

The source of hydrogen cyanide may be hydrogen cyanide gas itself, when the reaction is optionally performed in the presence of a base, for example pyridine; but it is preferably prepared in situ (to avoid the direct use of hydrogen cyanide) from a metal cyanide salt (generally an alkali metal cyanide, for example sodium cyanide or potassium cyanide),in the presence of an acid. Suitable acids include organic acids such as aliphatic carboxylic acids for example acetic acid, or halogenated aliphatic carboxylic acids for example chloroacetic acid or trifluoroacetic acid; sulphonic acids such as benzenesulphonic acid, 4-toluenesulphonic acid or methanesulphonic acid; or inorganic acids such as hydrochloric acid or sulphuric acid.

Alternative sources of hydrogen cyanide (which may be generated in situ) are trimethylsilylcyanide in water, or a mixture of trimethylsilylcyanide and a Lewis acid, for example tin (IV) tetrachloride, in a solvent such as dichloromethane or tetrahydrofuran, at a temperature of from 20°C to 100°C, preferably from 30°C to 60°C. The reaction is preferably performed under increased pressure which increases the speed of the reaction.

The preparation of compounds of formula (V) from compounds of formula (I) may be effected in a polar or a non-polar solvent. Examples of polar solvents which may be used include water; alcohols such as methanol or ethanol; N,N-dimethylformamide; dimethylsulphoxide; or alkanoic acids such as acetic acid. Examples of non-polar solvents include hydrocarbons such as hexane, or ethers such as tetrahydrofuran, dioxane or dialkyl ethers such as diethyl ether; or nitriles such as acetonitrile.When a metal cyanide salt is used in the presence of an acid, the preferred solvent is water or a mixture of water with a water miscible solvent. An equimolar amount or excess of the cyanide source may be employed, generally from 1 to 4 molar equivalents are used. The reaction temperature is generally from 0°C to 100°C, preferably from 20°C to 50°C.

Most preferably the compound of formula (V) is 2-(2,6-dichloro-4-trifluoromethylphenylhydrazino)succinonitrile.

The process of the invention for the preparation of compounds of formula (V) represents in certain aspects an improvement over the prior art.

According to a further feature of the invention processes (A) and (C) can be combined to prepare a compound of formula (V) from a compound of formula (III).

According to a further feature of the invention processes (B) and (C) can be combined to prepare a compound of formula (V) from a compound of formula (III).

The compounds of formula (I) obtained by process (A) or (B) of the invention may be used in the preparation of pesticidally active 5-amino-1-arylpyrazole derivatives of formula (VI) according to the following reaction scheme: 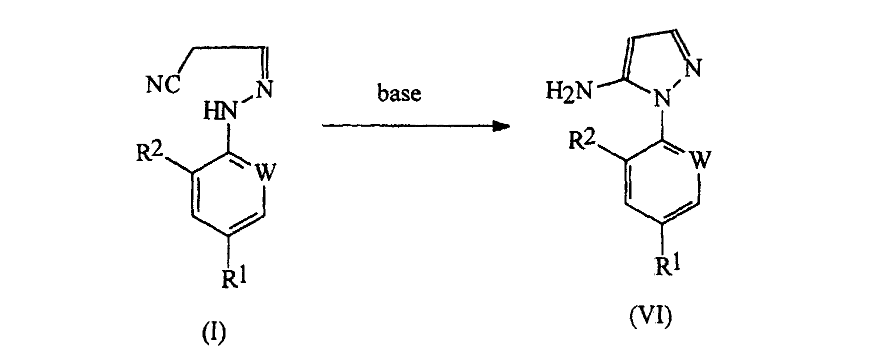 wherein R¹, R² and W are as hereinbefore defined.

The compounds of formula (V) obtained by the process (C) of the invention are particularly useful in the preparation of pesticidally active 5-amino-1-aryl-3-cyanopyrazole derivatives of formula (VII) obtained from intermediate compounds of formula (VIII) and (IX) according to the following reaction scheme: 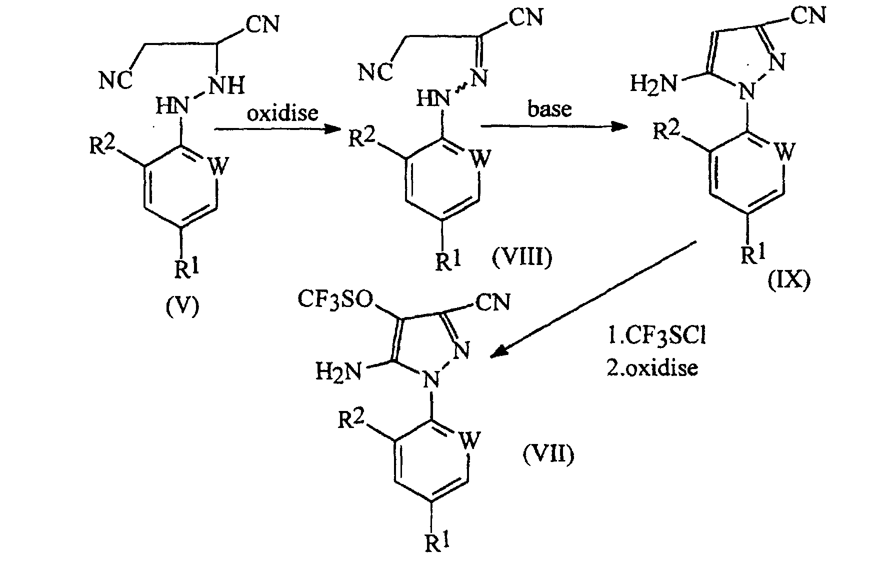 wherein R¹, R² and W are as hereinbefore defined.

The above processes for the preparation of compounds of formula(V) according to the invention, in combination with the above reaction steps for conversion of compounds of formula (V) into compounds of formula (VIII) and (IX) provide in certain aspects an unexpected improvement over the prior art.

Compounds of formula (VIII) may be prepared by the oxidation of compounds of formula(V). Suitable oxidants for the reaction include quinones such as benzoquinone, peroxides such as hydrogen peroxide, hypohalites such as sodium hypochlorite, or an alkali metal hydroxide such as sodium hydroxide in the presence of air, or preferably a metal salt or oxide for example cupric chloride or mercuric oxide. The reaction is generally conducted in a solvent. Solvents suitable for use include aromatic halogenated or non-halogenated hydrocarbons such as toluene or chlorobenzene, nitriles such as acetonitrile or amides such as N,N-dimethylformamide. The reaction temperature is generally from about 20 to about 150°C, and preferably from about 50 to about 100°C. The molar ratio of oxidant to compound of formula (V) is generally from 0.01:1 to 5:1, preferably from 1:1 to 3:1.

Compounds of formula (IX) may be prepared from compounds of formula (VIII) according to known methods.

The following non-limiting examples illustrate the invention. NMR spectra are recorded using deuterochloroform as solvent.

### Example 1

### Preparation of 3-(2,6-dichloro-4-trifluoromethylphenylhydrazono)propionitrile from 3,3-dimethoxypropionitrile

2,6-Dichloro-4-trifluoromethylphenylhydrazine hydrochloride was prepared by bubbling hydrogen chloride gas into an ether solution of 2,6-dichloro-4-trifluoromethylphenylhydrazine and filtration of the hydrochloride salt which was obtained in quantitative yield. Carbon tetrachloride (5ml) and 3,3-dimethoxypropionitrile (141 microlitres) were added successively to a solution of the above 2,6-dichloro-4-trifluoromethylphenylhydrazine hydrochloride (0.349g) in water (5ml) was heated at 75°C for 10 hours. The cooled mixture was extracted (dichloromethane), washed (water), dried (magnesium sulphate) and evaporated to give the title compound (0.358g), NMR 3.37(d,2H), 7.03(t,1H), 7.5(s,2H), 7.75(s,1H). The yield was 98%.

### Example 2

### Preparation of 3-(2,6-Dichloro-4-trifluoromethylphenylhydrazono)propionitrile from 3,3-dimethoxypropionitrile

A mixture of 2,6-dichloro-4-trifluoromethylphenylhydrazine (1.8g) and hydrochloric acid (4ml of 2N, 1 equivalent) was heated to 80°C under an inert atmosphere. 3,3-Dimethoxypropionitrile (912 microlitres, 1 equivalent) was added in one portion and the mixture heated at 80°C for 2 hours, cooled, extracted (dichloromethane), washed (water), dried (magnesium sulphate) and evaporated. The residue was purified by chromatography on silica gel eluting with dichloromethane to give the title compound (1.4g), NMR 3.37(d,2H), 7.03(t,1H), 7.5(s,2H), 7.75(s,1H). The yield was 59%.

### Example 3

### Preparation of 3-(2,6-Dichloro-4-trifluoromethylphenylhydrazono)propionitrile from 3-methoxy-acrylonitrile

By proceeding according to Example 1 but replacing the 3,3-dimethoxypropionitrile by 3-methoxy-acrylonitrile there was obtained, after purification by chromatography on silica gel eluting with dichloromethane, the title compound, NMR 3.37(d,2H), 7.03(t,1H), 7.5(s,2H), 7.75(s,1H). The yield was 63%.

### Example 4

### Preparation of 2-(2,6-dichloro-4-trifluoromethylphenylhydrazino)succinonitrile.

2-(2,6-Dichloro-4-trifluoromethylphenylhydrazono)succinonitrile (0.296g, lmmol), sodium cyanide (0.196g, 4 equivalents), water (1ml) and acetic acid (5ml) were added successively to a sealed tube. After reacting for 40 hours at 20°C the mixture was added to saturated sodium bicarbonate solution, extracted (dichloromethane), washed (water), dried (magnesium sulphate) and evaporated to give a mixture which contained 40% of the desired title compound, NMR 3.1 (m,2H), 4.5(m,1H), 5.89(m, 1H), 6.94(d,1H), 7.71(s,2H), together with 60% of unchanged starting hydrazone.

### Reference Example

### i) Preparation of 2-(2,6-dichloro-4-trifluoromethylphenylhydrazono)succinonitrile

A mixture of 2-(2,6-dichloro-4-trifluoromethylphenylhydrazino)succinonitrile (0.323g) and cupric chloride (0.175g) was heated in chlorobenzene at 60°C for 6 hours. After filtration and evaporation the title compound and 5-amino-3-cyano-1-(2,6-dichloro-4-trifluoromethylphenyl)pyrazole were obtained as a 7:1 mixture. Column chromatography on silica gel eluting with dichloromethane gave the pure title compound, obtained as a mixture of syn and anti isomers, NMR (anti isomer) 3.6(s,2H), 7.57(s,2H), 8.82(s,1H, exchangeable with D₂O), NMR (syn isomer) 3.56(s,2H), 7.59(s,2H), 8.27(s,1H,exchangeable with D₂O).

### ii) Preparation of 5-amino-3-cyano-1-(2,6-dichloro-4-trifluoromethylphenyl)pyrazole

Ammonia (20 microlitres of an 8% ammonia solution in water) was added to a mixture of the above 2-(2,6-dichloro-4-trifluoromethylphenylhydrazono)-succinonitrile (0.077g) in ethanol (1ml) and water (0.2ml) at 0 °C. After 10 minutes the mixture was extracted (dichloromethane) and evaporated to give 5-amino-3-cyano-1-(2,6-dichloro-4-trifluoromethylphenyl)pyrazole (0.076g, 97% yield). Purity 98% (by hplc).

## Claims

1. A process for the preparation of a compound of forumula (1): 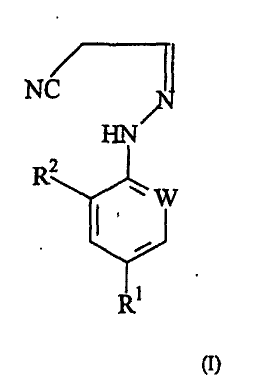 wherein
W represents nitrogen or -CR³;
R¹ represents halogen, haloalkyl (preferably trifluoromethyl), haloalkoxy (preferably trifluoromethoxy), R⁴S(O)n-, or -SF₅;
R² represents hydrogen or halogen (for example chlorine or bromine);
R³ represents halogen (for example chlorine or bromine);
R⁴ represents alkyl or haloalkyl; and
n represents 0,1 1 or 2; which process comprises the reaction of a compound of formula (II) or (IV): 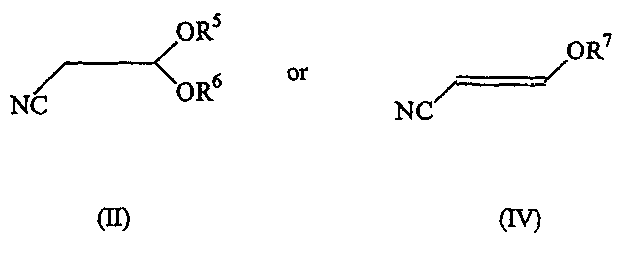
wherein R⁵ and R⁶ independently represent alkyl or together represent an alkylene chain containing two or three carbon atoms and R⁷ represents alkyl (preferably methyl or ethyl) with an acid addition salt of an arylhydrazine compound of formula (III): 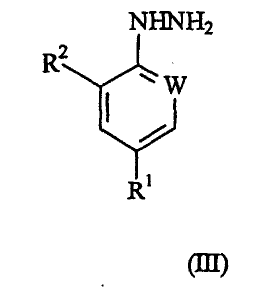 wherein R¹, R² and W are as defined in claim 1.

2. A process according to claim 1 in which R⁵ and R⁶ each represent methyl or ethyl.

3. A process according to claim 1 or 2 in which the acid addition salts of the compounds of formula (III) are the salts formed from strong acids.

4. A process according to any one of the preceding claims in which water is present.

5. A process according to any one of the preceding claims followed by the reaction of the compound of formula (I) obtained wherein R¹, R² and W are as defined in claim 1, with a source of hydrogen cyanide, to prepare a compound of formula (V): 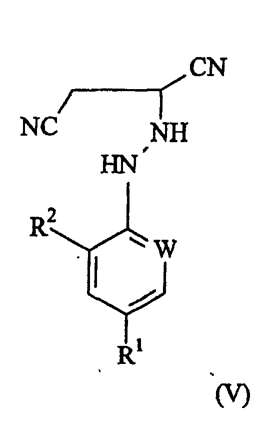 wherein R¹, R² and W are as defined in claim 1.

6. A process according to claim 6 in which the hydrogen cyanide is prepared from a metal cyanide salt in the presence of an acid.

7. A process according to claim 5 or 6 which is performed under increased pressure.

8. A process according to claim 5, 6 or 7 in which the compound of formula (V) obtained is oxidised and optionally reacted with a base to prepare a compound of formula (VIII) or (IX): 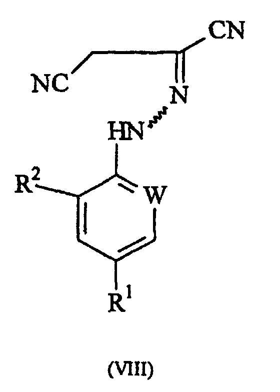 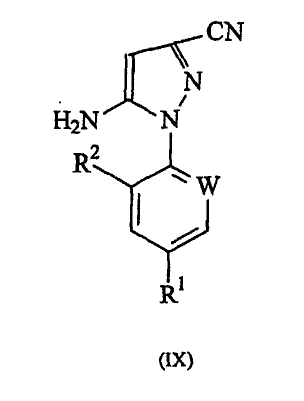 wherein W, R¹ and R² are as defined in claim 1.

9. A process according to any one of the preceding claims wherein R¹ represents haloalkyl (preferably trifluoromethyl), haloalkoxy (preferably trifluoromethoxy) or -SF₅;
W represents -CR³; and R³ represents halogen.

10. A process according to any one of the preceding claims wherein R¹ represents trifluoromethyl, W represents -CR³, and R² and R³ represent chlorine.

## Patentansprüche

1. Verfahren zur Herstellung einer Verbindung der Formel (I) 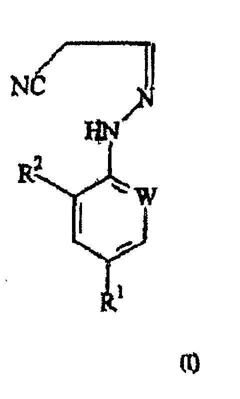 in der
W Stickstoff oder -CR³ bedeutet,
R¹ Halogen, Halogenalkyl (vorzugsweise Trifluormethyl), Halogenalkoxy (vorzugsweise Trifluormethoxy), R⁴S(O)n- oder -SF₅ bedeutet,
R² Wasserstoff oder Halogen (zum Beispiel Chlor oder Brom) bedeutet,
R³ Halogen (zum Beispiel Chlor oder Brom) bedeutet,
R⁴ Alkyl oder Halogenalkyl bedeutet, und
n 0, 1 oder 2 bedeutet,
**dadurch gekennzeichnet, daß** man eine Verbindung der Formel (II) oder (IV) 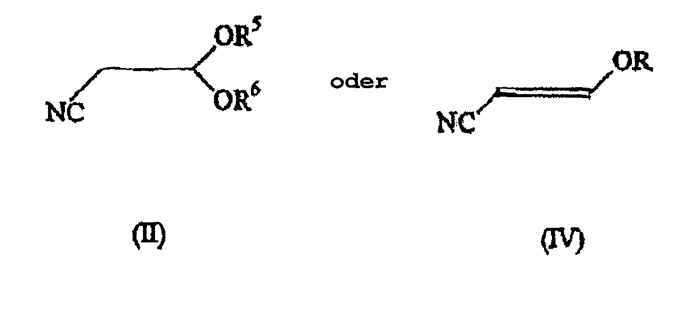 in denen R⁵ und R⁶ unabhängig voneinander Alkyl bedeuten oder gemeinsam eine Alkylenkette, die zwei oder drei Kohlenstoffatome enthält, bedeuten und R⁷ Alkyl (vorzugsweise Methyl oder Ethyl) bedeutet, mit einem Säureadditionssalz einer Arylhydrazinverbindung der Formel (III) 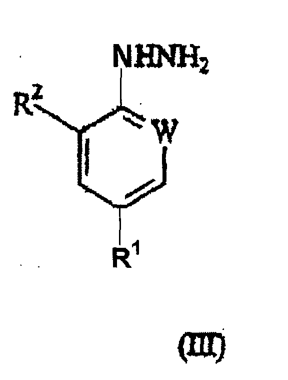 in der R¹, R² und W wie in Anspruch 1 definiert sind, umsetzt.

2. Verfahren nach Anspruch 1, wobei R⁵ und R⁶ jeweils Methyl oder Ethyl bedeuten.

3. Verfahren nach Anspruch 1 oder 2, wobei die Säureadditionssalze der Verbindungen der Formel (III) mit starken Säuren gebildete Salze sind.

4. Verfahren nach einem der vorhergehenden Ansprüche in der Gegenwart von Wasser.

5. Verfahren nach einem der vorhergehenden Ansprüche und anschließendes Umsetzen der erhaltenen Verbindung der Formel (I), in der R¹, R² und W wie in Anspruch 1 definiert sind, mit einer Cyanwasserstoffquelle zur Herstellung einer Verbindung der Formel (V) 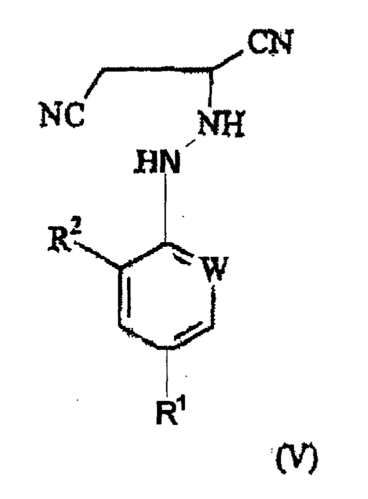 in der R¹, R² und W wie in Anspruch 1 definiert sind.

6. Verfahren nach Anspruch 6, wobei der Cyanwasserstoff aus einem Metallcyanidsalz in Gegenwart einer Säure hergestellt wird.

7. Verfahren nach Anspruch 5 oder 6, das unter erhöhtem Druck durchgeführt wird.

8. Verfahren nach Anspruch 5, 6 oder 7, bei dem zur Herstellung einer Verbindung der Formel (VIII) oder (IX), 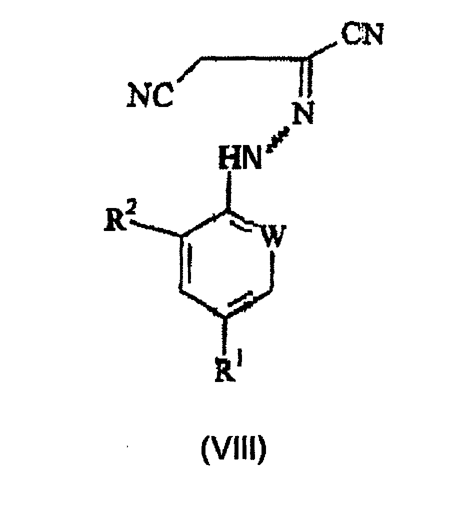 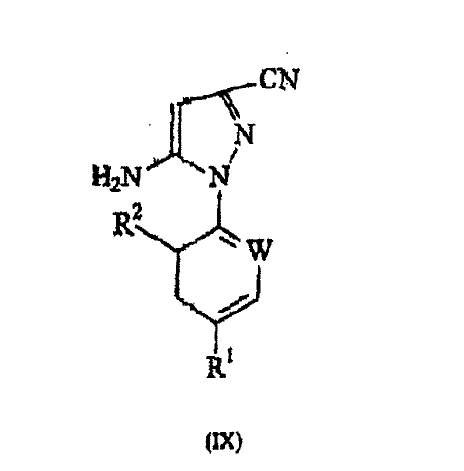 in der W, R¹ und R² wie im Anspruch 1 definiert sind, die erhaltene Verbindung der Formel (V) oxidiert und gegebenenfalls mit einer Base umgesetzt wird.

9. Verfahren nach einem der vorhergehenden Ansprüche, wobei R¹ Halogenalkyl (vorzugsweise Trifluormethyl), Halogenalkoxy (vorzugsweise Trifluormethoxy) oder -SF₅ bedeutet und
W -CR³ bedeutet und R³ Halogen bedeutet.

10. Verfahren nach einem der vorhergehenden Ansprüche, wobei R¹ Trifluormethyl bedeutet, W -CR³ bedeutet, und R² und R³ Chlor bedeuten.

## Revendications

1. Procédé de préparation d'un composé de formule (I) : 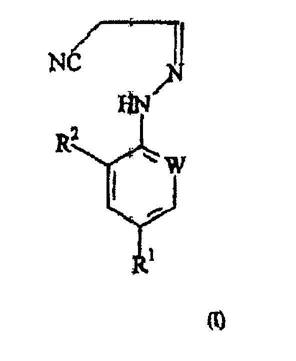 dans laquelle
W est azote ou -CR³ ;
R¹ représente halogéno, halogénoalkyle (de préférence trifluorométhyle), halogénoalcoxy (de préférence trifluorométhoxy), R⁴S(O)n-, ou -SF₅ ;
R² représente hydrogène ou halogène (par exemple chlore ou brome) ;
R³ représente halogène (par exemple chlore ou brome) ;
R⁴ représente alkyle ou halogénoalkyle ; et
n représente 0, 1 ou 2 ; lequel procédé comprend la réaction d'un composé de formule (II) ou (IV) : 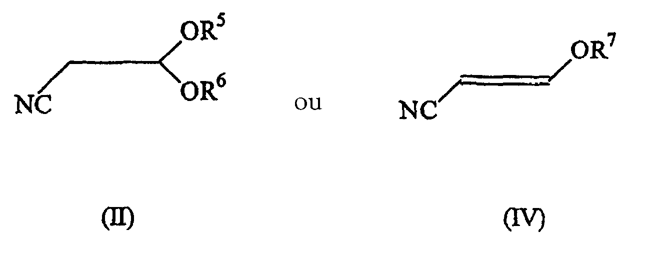
dans laquelle R⁵ et R⁶ représentent indépendamment alkyle ou représentent conjointement une chaîne alkylène contenant deux ou trois atomes de carbone et R⁷ représente alkyle (de préférence méthyle ou éthyle.) avec un sel d'addition d'acide d'un composé arylhydrazine de formule (III) : 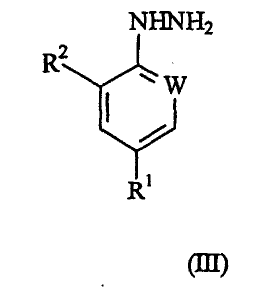 dans laquelle R¹, R² et W sont tels que définis dans la revendication 1.

2. Procédé selon la revendication 1, **caractérisé en ce que** R⁵ et R⁶ représentent chacun méthyle ou éthyle.

3. Procédé selon la revendication 1 ou 2, **caractérisé en ce que** les sels d'addition d'acides de composés de formule (III) sont les sels formés avec les acides forts.

4. Procédé selon l'une quelconque des revendications précédentes, **caractérisé en ce que** l'eau est présente.

5. Procédé selon l'une quelconque des revendications précédentes, **caractérisé en ce qu'**il est suivi de la réaction du composé de formule (I) obtenu, dans laquelle R¹, R² et W sont tels que définis dans la revendication 1, avec une source de cyanure d'hydrogène, pour préparer un composé de formule (V) : 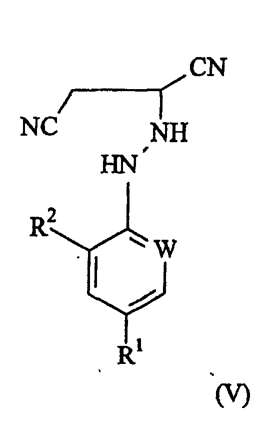 dans laquelle R¹, R² et W sont tels que définis dans la revendication 1.

6. Procédé selon la revendication 5, **caractérisé en ce que** le cyanure d'hydrogène est préparé à partir d'un sel de cyanure métallique en présence d'un acide.

7. Procédé selon la revendication 5 ou 6, **caractérisé en ce qu'**il est réalisé sous pression accrue.

8. Procédé selon la revendication 5, 6 ou 7, **caractérisé en ce que** le composé de formule (V) obtenu est oxydé et éventuellement mis en réaction avec une base pour préparer un composé de formule (VIII) ou (IX) : 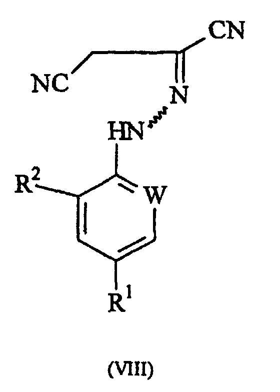 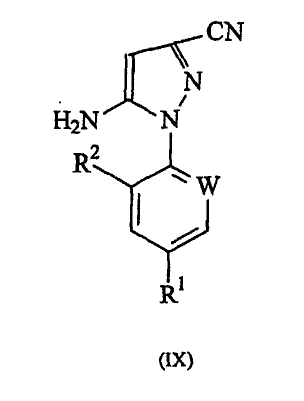 dans laquelle W, R¹ et R² sont tels que définis dans la revendication 1.

9. Procédé selon l'une quelconque des revendications précédentes, **caractérisé en ce que** R¹ représente halogénoalkyle (de préférence trifluorométhyle), halogénoalcoxy (de préférence trifluorométhoxy) ou -SF₅ ;
W représente -CR³; et R³ représente halogène.

10. Procédé selon l'une quelconque des revendications précédentes, **caractérisé en ce que** R¹ représente trifluorométhyle, W représente -CR³, et R² et R³ représentent chlore.
